# EUROPEAN PATENT APPLICATION

(11) **EP 2 659 925 A1**
(43) Date of publication of application: **06.11.2013**
(21) Application number: 12166362.9
(22) Date of filing: 02.05.2012
(51) Int. Cl.: A61M 5/315

(54) **Piston for a cartridge for use in a drug delivery device**

(71) Applicant: Sanofi-Aventis Deutschland GmbH, 65929 Frankfurt am Main (DE)
(72) Inventor: Otten, Martin Dl, 65926 Frankfurt am Main (DE); Weber, Lothar, 65926 Frankfurt am Main (DE)

(57) **Abstract**

The present invention relates to a piston for a cartridge (10) to be filled with a medicament (5), the piston comprising:
- a first piston member (16; 19; 26) comprising a first material providing a distal end face (15), and
- a second piston member (18; 28) comprising a second material and providing a proximal end face (17; 27) facing away from the distal end face (15; 25),
- wherein the second material comprises a lower compressibility compared to the first material. Moreover, the invention also relates to a cartridge and to a drug delivery device comprising such piston.

## Description

### Field of the Invention

The present invention relates to a piston for a cartridge at least partially filled with a medicament. In particular, the invention relates to pistons that serve as a seal for tubular shaped cartridges to be used in drug delivery devices such like pen-type injectors.

### Background and Prior Art

User operated drug delivery devices are as such known in the prior art. They are typically applicable in circumstances, in which persons without formal medical training, i.e., patients, need to administer an accurate and predefined dose of a medicinal product, such as heparin or insulin. In particular, such devices have application, where a medicament is administered on a regular or irregular basis over a short term or long-term period.

In order to accommodate with these demands, such devices have to fulfil a number of requirements. First of all, the device must be robust in construction, yet easy to use in terms of handling and in understanding by the user of its operation and the delivery of the required dose or medicament. The dose setting must be easy and unambiguous. Where the device is to be disposable rather than reusable, the device should be inexpensive to manufacture and easy to dispose.

Such devices, in particular pen-type injectors are typically adapted to receive a replaceable or disposable cartridge containing and providing the medicament to be disposed by the device. The cartridge comprises an outlet to be coupled with a piercing element, e.g. an injection needle, a cannula or the like in a fluid transferring way. Opposite to the distal dispensing end, the cartridge is typically sealed by way of a displaceable piston slidably arranged in the body of the cartridge. In order to expel a predefined dose of the medicament, a plunger or piston rod of a drug delivery device is adapted to act on the piston for displacing said piston by a predefined distance in distal, thus dose-dispensing direction.

On the one hand, the piston has to provide a durable and leak-proof seal in order to prevent any uncontrolled discharge of the medicament. On the other hand, the piston also has to prevent ingress of impurities or other external substances into the drug receiving volume of the cartridge. Apart from its sealing capability, the piston has to be displaceable with respect to the side walls of the cartridge. Friction forces inherently present at the contact surfaces of cartridge side walls and piston should be kept to a minimum to allow for an easy and smooth operation of the cartridge.

In particular when the device and/or its cartridge are used for the first time, the dosing accuracy may be suboptimal and hence crucial. Especially prior to an initial use of the device, its mechanically interacting components may not yet fully engage and/or mutual abutment of piston rod and piston of the cartridge is just obtained only during an initial distal displacement of the piston rod. In such situations, an initial dose dispensed by the device might be too small.

Moreover, since the piston sealing the vitreous and tubular shaped body of the cartridge is of a comparatively elastic and compressible material, the piston itself may experience a respective compression in axial direction during an initial or during repeated displacement of the piston rod. Such a compression may further sustain due to frictional forces between the inside facing side wall of the cartridge's body and the lateral or circumferential side wall or respective sealing lips of the piston.

Moreover, an eventual axial relaxation of the piston is rather disadvantageous and may effectuate an increase of a fluid pressure inside the cartridge. In situations where the cartridge is still in fluid connection with e.g. a piercing element, such internal pressure may lead to a post-dispensing droplet generation to be observed at a distal tip of the piercing assembly.

Document WO 2010/133675 A1 for instance discloses a bung for drug containing cartridges having a distal and a proximal end face. This bung also comprises at least two different materials, wherein a first material covers the whole lateral area of the bung and wherein a second material is at least partly arranged inside the bung. Furthermore, the first material has a larger compressibility than the second material.

Even though this bung provides a reduced axial compressibility, manufacturing of such a piston is cumbersome and cost-intensive since the second material is entirely surrounded by the first material. Manufacturing of such a bung may for instance require a multicomponent injection moulding process.

### Objects of the invention

It is therefore an object of the present invention to provide an improved piston for a cartridge to be used with a drug delivery device, wherein the piston comprises a reduced degree of compressibility, thereby providing improved dosing accuracy. Additionally, the piston should be easy to assemble in a cost-efficient way, preferably in a mass-production environment.

### Summary of the Invention

In a first aspect, the invention provides a piston for a cartridge to be filled with a medicament. The piston typically serves as a proximal seal of a tubular shaped barrel of the cartridge in order to hermetically seal the inner volume of said cartridge. Furthermore, the piston is adapted and designed to be displaced in axial direction with respect to the geometry of the cartridge, preferably by means of a piston rod or by way of a comparable driving member, e.g. of a drive mechanism of a drug delivery device.

Since the piston is sealingly engaged with the inside facing sidewalls of the cartridge, a distally directed displacement of the piston may lead to a respective built-up of a fluid pressure inside the volume of the cartridge for dispensing a well-defined amount of the medicament via a distal outlet end for instance.

Typically, the cartridge not only comprises the piston but also another, pierceable sealing member at a distal end by way of which the liquid medicament can be expelled from the cartridge in response to a distally directed displacement of the piston relative to the cartridge. The cartridge may be designed as a carpule or vial to be removably inserted into a drug delivery device, such like a pen-type injector.

In another aspect, the cartridge may also comprise or even constitute a pre-filled syringe having a piston slidably disposed therein.

Irrespective on the type of cartridge the piston is designed for, the piston comprises a first piston member comprising a first material and providing a distal end face of the piston. Additionally, the piston also comprises at least a second piston member comprising a second material and providing a proximal end face of the piston. Proximal and distal end faces are located on opposite ends of the piston and therefore face away from each other. While the proximal end face typically faces towards a piston rod to operably engage with the piston, the distal end face points towards the inner volume of the cartridge and hence towards the medicament disposed therein.

Consequently, the distal end face typically serves as sealing face whereas the proximal end face acts as thrust- or pressure receiving end of the piston.

The second material of which the second piston member is preferably made of comprises a lower compressibility than the first material, of which the first member is preferably made of. In other words, the material of the first piston member features a larger compressibility compared to the second material and therefore comprises a larger degree of elasticity compared to the second piston member. Accordingly, the second material features a larger degree of rigidity compared to the first piston member and may therefore provide mechanical strength and stability to the piston.

The second piston member may also provide enhanced stiffness to the piston and therefore allows to reduce the axial dimensions of the piston in its entirety. The second piston member of decreased compressibility and reduced elasticity therefore serves as a thrust receiving and thrust- or pressure transmitting component of the piston. It is therefore adapted to transmit and to apply mechanical forces and pressure to the first piston member in a rather unadulterated way.

In comparison to conventional piston designs, the axial dimensions of the first, rather soft and elastic piston member can be reduced while the axial elongation of the second, rather rigid piston member increases accordingly.

By reducing the axial dimensions of the rather elastic component of a piston, the axial compressibility of the entire piston can be reduced in order to achieve improvements in regard of dosing accuracy. In effect, elastic relaxation processes as well as storage of mechanical or elastic energy in a comparatively elastic piston material can be significantly reduced by making use of a rather hard and rigid material of the second piston member.

In a preferred embodiment, the first material, the first piston member is made of comprises an elastomeric material. Preferably, the first material comprises a natural and/or a synthetic rubber adapted to provide a sufficient sealing between the first piston member and the inner side wall structures of the piston. Preferably, the first material comprises butyl rubber such like bromobutyl-rubber.

In a further preferred embodiment, the second material comprises a plastic material, preferably a thermoplastic material, such like cyclic olefin copolymer (COT) or other thermoplastic materials, such like polypropylene, polyethylene, or the like. Since the second material does not get in direct contact with the medicament disposed in the cartridge, the material, the second piston member is made of can be almost arbitrarily chosen.

It is even conceivable, that the second material comprises a metal or a ceramic material.

In a further preferred aspect, the first and the second piston members comprise a substantially cylindrical shape and mutually abut in axial direction. First and second piston members comprise a somewhat identical or similar shape. Both piston members may be of tubular or disk-like shape and are adapted to mutually engage across an interface region extending in a lateral plane with respect to the substantially cylindrical or tubular geometry of the cartridge.

Additionally, the first piston member featuring pre-defined dimensions in order to precisely match with the cartridge might be used with a variety of different second piston members featuring different mechanical or geometric properties depending on the particular cartridge the piston is to be assembled in. Additionally, it is conceivable, that a first piston member of pre-defined type and/or dimensions is to be coupled with a variety of different second piston members in order to selectively modify the overall compressibility and geometry of the piston.

In a further preferred embodiment, the first and second piston members are mutually bonded or adhesively engaged. In this configuration it is even conceivable, that first and second piston members are mutually attached and interconnected by way of a single or multicomponent injection moulding process. However and according to another preferred embodiment, first and second piston members may also be positively engaged by making use of respective interconnecting members.

In effect, first and second piston members that may be manufactured separately are to be mutually assembled prior to a final assembly of the piston in the cartridge. Assembly of first and second piston members may be conducted by way of injection moulding but can also be performed all-mechanically in a separate piston assembly step by way of which the interconnecting members mutually engage.

Mutually corresponding and positively engaging first and second piston members are particularly suitable to provide a large variety of pistons featuring different axial dimensions and different degrees of mechanical compressibility.

In a further preferred aspect, first and second piston members are inseparably engaged so as to prevent a disassembly or disengagement once inserted and assembled in a cartridge.

In a further embodiment, the first piston member comprises a circumferential sealing lip so as to frictionally engage with lateral or circumferential inside facing side wall portions of the barrel of the cartridge. The circumferential lip of the first piston member may substantially flush in axial direction with the second piston member interconnected to the first piston member. It may be of further benefit, when the first piston member comprises at least one or several circumferential sealing lips spaced apart in axial direction so as to improve the hermetic seal between the piston and the barrel of the cartridge.

Alternatively, it is also conceivable, that the sealing lip of the first piston member radially protrudes from the lateral side wall portion of the second piston member which is disposed adjacent thereto.

The second piston member may comprise a rather rigid and solid material. In an alternative embodiment, the second piston member comprises a stiffening or structural enhancing mechanical structure of skeleton or framework-type. Thus, the second piston member may comprise a circumferential rim that matches and corresponds with e.g. the radially outwardly extending circumferential sealing lip of the first piston member. By providing a framework-type second piston member, the total volume and weight of such piston member can be advantageously reduced.

In a further embodiment, the second piston member also comprises at least one support structure extending across the rim and substantially lying in or overlapping with the lateral plane of the rim. Here, it is of further benefit when the second piston member comprises two or even more intersecting support structures which mutually cross or intersect. The various support structures may extend equidistantly with respect to the circumferential or tangential direction.

In a further preferred aspect, the axial elongation of the second piston member is at least equal or larger than the axial elongation of the first piston member. In terms of the axial direction, the first piston member may be rather thin compared to the second piston member. The first piston member may therefore only effectively provide a sealing layer or coating across the first piston member. It is of particular benefit, when the axial dimension of the first piston member is not more than 50 %, 40 %, 30 %, 20 % or even less than 10 % of the total axial elongation of the piston. Consequently, the axial elongation of the second piston member is at least 50 %, 60 %, 70 %, 80 % or even larger than 90 % of the total elongation of the piston.

In effect, axial elongation of the first piston member may only be reduced to such a minimum in which a sufficient sealing between the piston and the tubular barrel of the cartridge can still be provided.

In a further independent aspect, the invention also relates to a cartridge to be used as or in a drug delivery device. The cartridge comprises a tubular shaped barrel or a body at least partially filled with a medicament, preferably with a liquid medicament. The cartridge further comprises a piston as described above and is displaceably arranged in the barrel in an axial direction, preferably at least in distal direction in order to expel a predefined amount of the medicament.

In still another aspect, the invention also relates to a drug delivery device, such like a pen-type injector being equipped with the aforementioned cartridge and therefore comprising at least one of the above-described pistons.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2, des Pro36 Exendin-4(1-39),
des Pro36 [Asp28] Exendin-4(1-39),
des Pro36 [isoAsp28] Exendin-4(1-39),
des Pro36 [Met(O)14, Asp28] Exendin-4(1-39),
des Pro36 [Met(O)14, lsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(02)25, Asp28] Exendin-4(1-39),
des Pro36 [Trp(02)25, IsoAsp28] Exendin-4(1-39),
des Pro36 [Met(O)14 Trp(02)25, Asp28] Exendin-4(1-39),
des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two 13 sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem lg domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single lg domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be further apparent to those skilled in the art that various modifications and variations can be made to the present invention without departing from the spirit and scope of the invention. Further, it is to be noted, that any reference signs used in the appended claims are not to be construed as limiting the scope of the present invention.

### Brief Description of the Drawings

In the following, preferred embodiments of the invention will be described by making reference to the drawings, in which:
- Figure 1: schematically illustrates a two-component piston disposed in a tubular barrel of a cartridge,
- Figure 2a: schematically illustrates a cross section of a first embodiment of the piston,
- Figure 2b: shows another embodiment of the piston in cross section
- Figure 2c: depicts a further embodiment of the piston in cross section;
- Figure 2d: illustrates another embodiment of the piston in cross section,
- Figure 3: shows a side view of another piston featuring a radially protruding sealing lip,
- Figure 4: shows individual interconnecting members of the first piston member and
- Figure 5: schematically illustrates a framework-like embodiment of a second piston member.

### Detailed Description

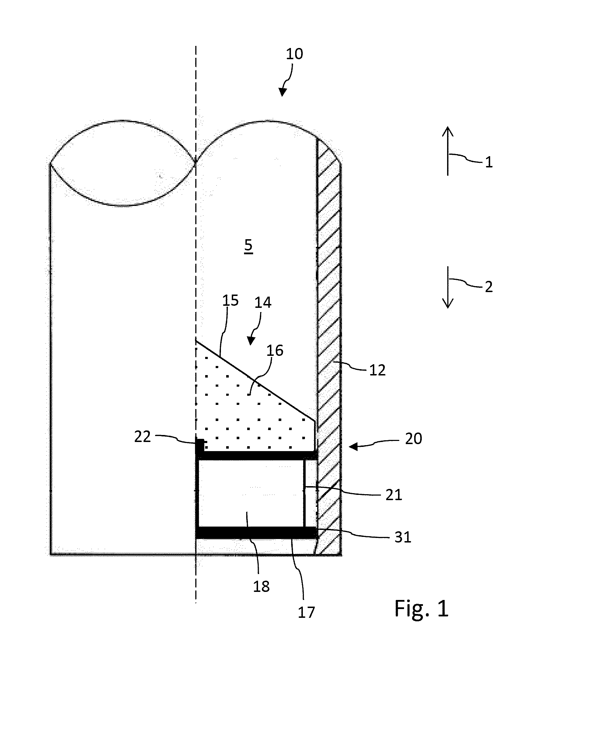

In Figure 1, there is schematically illustrated a cartridge 10 featuring a barrel 12 of substantially tubular shape and extending in axial direction, hence in distal direction 1 and in opposite, proximal direction 2. Inside the cartridge 10 there is disposed a piston 14 comprising a first piston member 16 and a second piston member 18. First and second piston members 16, 18 are substantially equal in shape, at least with respect to their radial or transverse dimensions. First and second piston members 16, 18 are mutually interconnected by some kind of interface 20 that extends in the lateral or transverse plane of the piston 14.

The first piston member 16 provides a distal end face 15 of the piston 14 which may get in contact with a medicament 5 disposed in a distal portion of the barrel 12. The second piston member 18 may be in direct contact with and/or may extend across a proximal end face of the first member 16 and may therefore comprise a proximal end face 17 facing away from the first piston member 16. The proximal end face 17 may serve as a thrust receiving surface, e.g. when the piston 14 is mechanically engaged with a piston rod, which is not particularly illustrated here. The proximal end face 17 of the piston 14 may particularly serve as a thrust- or pressure receiving surface, in particular during a distally directed displacement of the piston member 14 relative to the cartridge 10 and relative to its barrel 12.

It is of particular benefit, that the material the first piston member 16 is made of comprises a larger compressibility compared to the material of which the second piston member 18 is made. In other words, the second piston member 18 features a lower compressibility compared to the first piston member 16. The second piston member 18 may therefore enhance and increase mechanical stability, rigidity and stiffness of the piston 14 in its entirety.

Preferably, the second piston member 18 is made of a comparatively hard plastic whereas the first piston member 16 comprises an elastomeric material, such like a synthetic or natural rubber inherently providing a sufficient sealing effect towards the lateral side wall portions of the tubular shaped barrel 12.

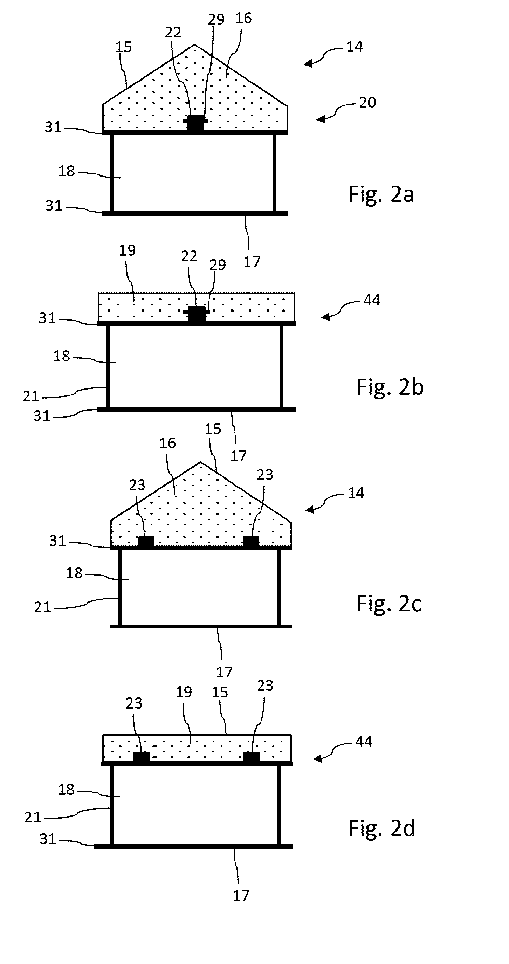

The two-component piston 14 comprises two piston members 16, 18, that may be mutually bonded, adhesively attached or frictionally engaged. It is also conceivable that the two piston members 16, 18 are positively engaged, e.g. by means of at least one interconnecting member 22 as for instance indicated in the various cross-sectional illustrations of Figure 2a to 2d.

According to the cross sectional illustration of the piston 14 in Figure 2a, the second piston member 18 comprises a sealing lip 31 radially extending from a lateral sidewall 21 of the second piston member 18. The sealing lip 31 is arranged in or forms the interface 20 between the first and second piston member 16, 18. The second piston member 18 further comprises at least one centrally located interconnecting member 22 extending in distal direction 1 and positively engaging with the first piston member 16.

As further illustrated in Figure 2a, the interconnecting member 22 also comprises radially extending barbs 29 by way of which a non-detachable interconnection between first and second piston members 16, 18 can be established. It is particularly intended that the radially extending barbs 29 are surrounded and/or embedded in the material of the first piston member 16, which may be molded around the interconnecting member 22 and its barbs 29.

As further indicated in Figures 1, 2a and 2c, the first piston member 16 comprises a somewhat tipped or slanted distal end face 15 extending from a central area radially outwardly in proximal direction 2. Here, the bevelled end face 15 may accommodate to the geometry and shape of a distally located end section of the barrel 12, which is not further illustrated here.

Figure 2b shows cross section of different shaped piston 44 comprising a different kind of a first piston member 19, which is rather slab-like and comprises a substantially rectangular cross section in the plane defined by axial and radial directions. The second piston members 18 of piston 14 and piston 44 are substantially identical and may be therefore universally utilized to manufacture different kinds of pistons 14, 44. Mutual fastening of first and second piston members 19, 18 of pistons 14 and 44 as shown in Figures 2a and 2b is somewhat identical and standardized.

The general shape of the piston 14 and its first piston member 16 as shown in Figure 2c is similar or almost identical to the one as shown in Figure 2a, except that the second piston member 18 comprises two distally extending interconnecting members 23 which are separated from each other in radial direction. Preferably, such interconnecting members 23 are regularly arranged across the interface 20 between first and second piston members 16, 18. They may either provide a positively-engaged or frictionally engaged interconnection of first and second piston members 16, 18. Moreover, the interconnecting members 23 may engage with correspondingly shaped recesses provided in the proximal end face of the first piston member 16.
In general, it is also conceivable, that the first piston member 16 comprises at least one or several proximally extending interconnecting members that match with correspondingly shaped recesses on a distal end face of the second piston member 18.

Figure 2a is further indicative of another embodiment, wherein the first piston member 19 as shown Figure 2b is interconnected with the second piston member 18 by means of several radially distributed interconnecting members 23.

Mutually corresponding connecting members 22, 23 of the first piston member 16 and the second piston member 18 may comprise mutually corresponding protrusions and recesses extending in axial direction 1, 2. Moreover, the interconnecting members 22 may comprise undercuts or similar indentations as well as respective catching and latching members. This way, the first piston member 16 and the second piston member 18 can be manufactured separately and can be mutually interconnected and engaged by way of an intermediate step of assembly.

Figures 2a to 2d further illustrate that various different types of first piston members 16, 19 can be variably interconnected with one or different types of a second piston member 18. In particular when having somewhat standardized interconnecting members, a whole series of different shaped and individually adapted pistons can be manufactured on the basis of a limited number of piston members 16, 18, 19. For instance, four different pistons can be manufactured or assembled on the basis of two different types of first and second piston members, respectively.

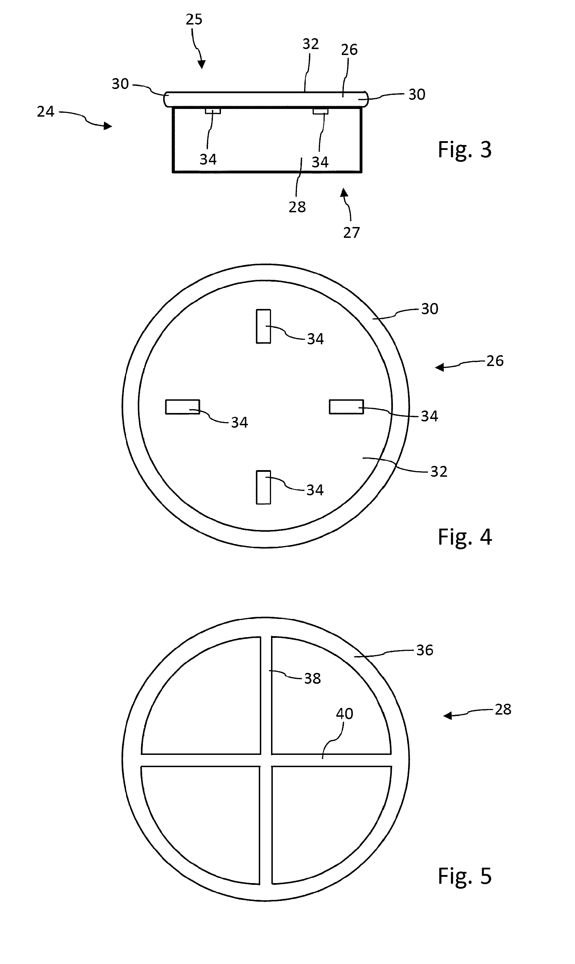

As further shown in the cross-sectional or side views of Figs. 2a to 2d and Figure 3, the first and the second piston members 16, 18 do not mutually intersect in axial direction but mutually abut in a plane extending substantially perpendicular to the axial direction 1, 2. Hence, first and second piston members 16, 18 may mutually abut and contact each other along a substantially flat- or even shaped contact surface.

The alternative embodiment of a piston 24 as indicated in Figure 3 comprises a first piston member 26 and a second piston member 28, wherein the axial extension of the first piston member 26 is substantially smaller than the axial extension of the second piston member 28. Also here, the first piston member 26 provides a distal end face 25 facing towards the medicament 5 contained in the cartridge 10. Accordingly, the second piston member 28 features a proximal end face 27 that serves as a thrust receiving surface for a distally directed externally driven displacement of the piston 24 inside the cartridge 10.

The first piston member 26 comprises a sealing surface 32 and comprises a circumferential sealing lip 30 extending radially outwardly. As shown in Figure 3, the first piston member 26 radially extends beyond the second sealing member 28 with its sealing lip 30. Even though a rather flat and even shaped sealing surface 32 is shown in Figure 3, the first piston member 16, 26 may also feature a curved, e.g. convex or spherical shaped sealing surface as for instance indicated with the piston 14 as shown in Figure 1.

In proximal direction 2 facing away from the distal sealing surface 32, the first piston member 26 comprises numerous interconnecting members 34 adapted to provide an interconnection with the second piston member 28, which, according to the illustration of Figure 5, comprises a framework-like structure.

Here, the second piston member 28 comprises a circumferential rim 36, which substantially matches and corresponds with the lateral sealing lip 30 of the first piston member 26. Additionally, the second piston member 28 comprises two support structures 38, 40 extending substantially perpendicular with respect to each other and extending across the circular structure of the circumferential rim 36. In the illustrated embodiment, the second piston member 28 serves to enhance rigidity and mechanical stability of the comparatively elastic and deformable first piston member 26. Furthermore, the interconnecting members 34 provided on the first piston member 26 are adapted to engage with corresponding interconnecting members located at the support structures 38, 40 of the second piston member 28.

### List of Reference Numerals

- 1: distal direction
- 2: proximal direction
- 5: medicament
- 10: cartridge
- 12: barrel
- 14: piston
- 15: distal end face
- 16: first piston member
- 17: proximal end face
- 18: second piston member
- 19: first piston member
- 20: interface
- 21: side wall
- 22: interconnecting member
- 23: interconnecting member
- 24: piston
- 25: distal end face
- 26: first piston member
- 27: proximal end face
- 28: second piston member
- 29: barb
- 30: sealing lip
- 31: sealing lip
- 32: sealing surface
- 34: interconnecting member
- 36: rim
- 38: support structure
- 40: support structure
- 44: piston

## Claims

1. A piston for a cartridge (10) to be filled with a medicament (5), the piston comprising:
- a first piston member (16; 19; 26) comprising a first material providing a distal end face (15), and
- a second piston member (18; 28) comprising a second material and providing a proximal end face (17; 27) facing away from the distal end face (15; 25),
- wherein the second material comprises a lower compressibility compared to the first material.

2. The piston according to claim 1, wherein the first material comprises an elastomeric material.

3. The piston according to claim 2, wherein the first material comprises a natural and/or a synthetic rubber.

4. The piston according to any one of the preceding claims, wherein the second material comprises a plastic material.

5. The piston according to any one of the preceding claims, wherein the first and the second piston members (16; 19; 26, 18; 28) comprise a substantially cylindrical shape and mutually abut in axial direction (1, 2).

6. A piston according to any one of the preceding claims, wherein the first piston member (16; 19; 26) and the second piston member (18; 28) are mutually bonded.

7. The piston according to any one of the preceding claims, wherein the first piston member (16; 19; 26) and the second piston member (18; 28) are positively engaged.

8. The piston according to any one of the preceding claims, wherein the first piston member (16; 19; 26) and the second piston member (18; 28) are inseparably engaged.

9. The piston according to any one of the preceding claims, wherein the first piston member (16; 19; 26) and/or the second piston member (18; 28) comprises at least one circumferential sealing lip (30; 31).

10. The piston according to any one of the preceding claims, wherein the second piston member (28) comprises a circumferential rim (36).

11. The piston according to claim 10, wherein the second piston member (28) comprises at least one support structure (38, 40) extending across the rim (36).

12. The piston according to any one of the preceding claims, wherein the axial elongation of the second piston member (18; 28) is at least equal or larger than the axial elongation of the first piston member (16; 26).

13. A cartridge to be used as or with a drug delivery device having a tubular shaped barrel (12) at least partially filled with a medicament (5) and comprising a piston (14; 24; 44) according to any one of the preceding claims and being displaceably arranged in the barrel (12) in an axial direction (1, 2).

14. A drug delivery device comprising a cartridge according to claim 13.
